(19) Europäisches Patentamt
European Patent Office
Office européen des brevets

(11) EP 0 565 542 B1

## (12) EUROPEAN PATENT SPECIFICATION

(45) Date of publication and mention
of the grant of the patent:
**21.08.1996 Bulletin 1996/34**

(21) Application number: **92901189.8**

(22) Date of filing: **22.11.1991**

(51) Int. Cl.$^6$: **A61F 2/06**

(86) International application number:
**PCT/US91/08587**

(87) International publication number:
**WO 92/11824 (23.07.1992 Gazette 1992/19)**

(54) **RESECTABLE SELF-EXPANDING STENT**

RESEKTIERBARER SELBSTAUSBREITENDER STENT

ELEMENT ATTELLE RESECABLE A AUTODILATATION

(84) Designated Contracting States:
**AT BE CH DE DK ES FR GB GR IT LI LU NL SE**

(30) Priority: **04.01.1991 US 637356**

(43) Date of publication of application:
**20.10.1993 Bulletin 1993/42**

(73) Proprietor: **American Medical Systems, Inc.
Minnetonka Minnesota 55345 (US)**

(72) Inventors:
• **BURTON, John, H.
Minnetonka, MN 55343 (US)**
• **STAEHLE, Bradford, G.
Minnetonka, MN 55343 (US)**
• **TIHON, Claude
Eden Prairie, MN 55347 (US)**

• **MIKULICH, Michael A.
CH-1093 La Conzersion (CH)**

(74) Representative: **Hayles, James Richard et al
Pfizer Limited,
Patents Department,
Ramsgate Road
Sandwich Kent CT13 9NJ (GB)**

(56) References cited:
| | |
|---|---|
| EP-A- 0 274 846 | EP-A- 0 382 014 |
| FR-A- 1 602 513 | US-A- 4 820 298 |
| US-A- 4 878 906 | US-A- 4 944 746 |

Remarks:
The file contains technical information submitted after the application was filed and not included in this specification

## Description

Background of the Invention

This invention relates to a stent according to the preamble of Claim 1. Such a stent is known e.g. from FR-A-1602513. Stent devices of this type are intended to be inserted in tubular body organs for maintaining the organ in a patent condition. The invention refers more particularly to the design of a tubular stent whose thermoplastic material and geometry allow it to expand by itself from a radially compressed condition to a larger diameter and which can later be resected using an electrosurgical instrument.

Various forms of surgical stents are known in the art for maintaining a tubular body organ, such as a vein, artery, bile duct, fallopian tube or urethra, in a patent condition whereby body fluids can continue to flow in a normal fashion. Consider the condition termed benign prostatic hypertrophy where, in the male urinary system, with age, the prostate gland may swell. If the urethra which the gland surrounds is collapsed to the point where the flow or urine from the bladder becomes partially or even fully blocked, surgical intervention is often required. In surgically addressing this problem, a transurethral resection of the prostate is often performed in which portions of the prostate gland are shaved or resected away using an electrosurgical instrument called a resectoscope.

Another approach in treating an enlarged prostate involves inserting a dilatation catheter into the urethra and advancing that catheter until the balloon portion thereof is aligned with the prostate. Then the balloon is inflated to stretch and enlarge the urethra. Another treatment involves the insertion of a stent which functions to re-enforce the urethra at the site so that the tissue involved does not collapse to obstruct urine flow.

Where a stent is to be implanted transurethrally, it is an important characteristic that it possess a low cross-sectional profile to facilitate its being routed to the desired site within the urethra. Once appropriately positioned, it is desirable that the stent expand to a larger diameter and that it remain stable at that diameter over an extended period to provide the necessary support for inhibiting the urethra from again collapsing. Various devices having this property are described in the patent art. For example, in U.S. Patent No. 4,655,771 to Wallsten, and in FR-A-1602513 and FR-A-2525896 there is described a tubular stent formed from braided metal wire which, when stretched longitudinally, will assume a relatively small diameter, but when it is allows to spring back to a shorter length, an attendant increase in the diameter takes place. These devices suffer from a number of practical problems, not the least of which is the difficulty in properly positioning the stent so that, when released, it will collapse longitudinally and increase in size radially to the point where patency is established along the length of the prostate without having a portion of the stent protrude into the external sphincter so as to result in urinary incontinence or, alternatively, into the bladder where it would serve as a nidus for stone formation.

Also, if a stent of the type described in the Wallsten patent remains in the body for a period of several months, tissue ingrowth occurs and the stent, because of its open construction, becomes incorporated into the vessel wall where it is shielded from the urine. However, should it become necessary to explant the stent for any reason, it becomes extremely difficult to remove it through the urethra.

U S Patent 4,893,623 (to Rosenbluth) describes a tubular stent where the wall of the tube is slit in a predetermined fashion. To implant the stent, it is mounted over a deflated balloon on a dilatation catheter and then routed to the appropriate site in the tubular organ where the stent is to be deployed. The stent is made from a malleable metal so that, when the balloon is inflated, it will stretch the walls of the stent, creating an open lattice pattern. When the balloon is again deflated, the stent will remain stretched to the diameter established by the inflated balloon and the dilatation catheter can again be withdrawn from the body.

The stent arrangement described in the Rosenbluth patent also becomes difficult to remove once tissue ingrowth has occurred. Moreover, it is not self-expanding but, instead, must be stretched to a desired diameter through the application of an outward radial force. When that outside force is removed, the stent does not provide a residual outward radial force against the vessel wall. This may lead to undesired migration of the stent within the hollow vessel subsequent to its implantation and prior to the establishment of tissue ingrowth.

It is accordingly a principal object of the present invention to provide an improved tubular stent for use in the lumen of a tubular body organ.

Another object of the invention is to provide a tubular stent which may readily be resected from a tubular body organ at a later time if deemed necessary.

Yet another object of the invention is to provide a self-expanding tubular stent which is capable of being inserted through the lumen of a tubular body organ while exhibiting a small diameter, but which is self-expanding upon being released from its insertion tool and which continues to exert a residual outward radial force against the vessel wall to maintain the stent in place.

A further object of the invention is to provide a tubular stent fabricated from a thermoplastic material, which is self-expanding and which is capable of being resected by being cut into pieces with an electrosurgical instrument.

Summary of the invention

According to the present invention, there is provided a stent for insertion into a tubular organ for maintaining the organ patent, comprising a single-piece tubular member consisting of a non-braided web or mesh formed into a closed tube of continuous section and without any overlapping longitudinal edges, having a side wall exhibiting a pattern of spaced openings defined by strands, said tubular member having a single-plane fenestrated side wall, with said side wall including a pattern of regular generally eye-shaped cutouts forming said spaced openings to allow said tubular member to be radially compressed from a larger diameter to a smaller diameter and which tubular member self-expands when the radial compressive force is removed, characterized in that said strands are intersecting strands integrally joined together at their points of intersection, the compression and later expansion not producing relative movement of said strands at said points of intersection or a significant change in axial length of said tubular member. It is preferably fabricated from a thermoplastic material, allowing same to be shaved or resected into smaller pieces for later removal should that become necessary. By controlling the electrical conductivity of the thermoplastic material so that it approximates that of human tissue, the ability to resect the stent using an electrosurgical instrument is enhanced.

The stent of the present invention comprises a non-braided thermoplastic web or mesh formed into a closed tube where the web or mesh includes a pattern of apertures of a predetermined shape that allows the closed tube to be radially compressed from a relatively larger diameter to a significantly smaller diameter when subjected to inward radially directed compressive forces uniformly applied over its surface, but which returns to a predetermined intermediate diameter when those compressive forces are removed. The intermediate diameter is sufficiently large to assure continuing outward force against the lumen wall. This tends to prevent unwanted migration prior to the time that tissue ingrowth occurs. A particularly efficacious device has been found to result with a pattern of openings defined by thin strands of DELRIN® plastic, an acetal homopolymer thermoplastic resin, whose radial thickness is about $1\frac{1}{4}$ to $2\frac{1}{4}$ times their circumferential width. With this pattern, the fenestrated tube may be radially compressed from a larger diameter, $d_1$, to a smaller diameter, $d_2 = d_1/4$. The ability of the stent to spring back to a predetermined outer diameter depends upon the degree of plastic deformation that the material incurs as well as the amount of creep encountered.

The features and advantages as well as the method of making and using the tubular stent of the present invention will become apparent to those skilled in the art from the following detailed description of a preferred embodiment, especially when considered in conjunction with the accompanying drawings in which numerals in the several views refer to corresponding parts.

Brief Description of the Drawings

Fig. 1 is a greatly enlarged perspective view of a self-expanding tubular stent (not in accordance with the present invention, but included to illustrate it);
Fig. 2 is a side elevation view of the stent of Figure 1 at the time of manufacture and prior to being loaded into the stent delivery device;
Fig. 3 is a side elevation view of the stent of Figure 1 when radially compressed for insertion into the lumen of a tubular body organ;
Fig. 4 is a side elevation view of the stent of Figure 1 following release from its insertion tool; and Fig. 5 is a side elevation view of a stent according to the invention having a pattern of apertures whose shape enhances the self-expanding characteristics of the device.

Detailed Description of the Invention

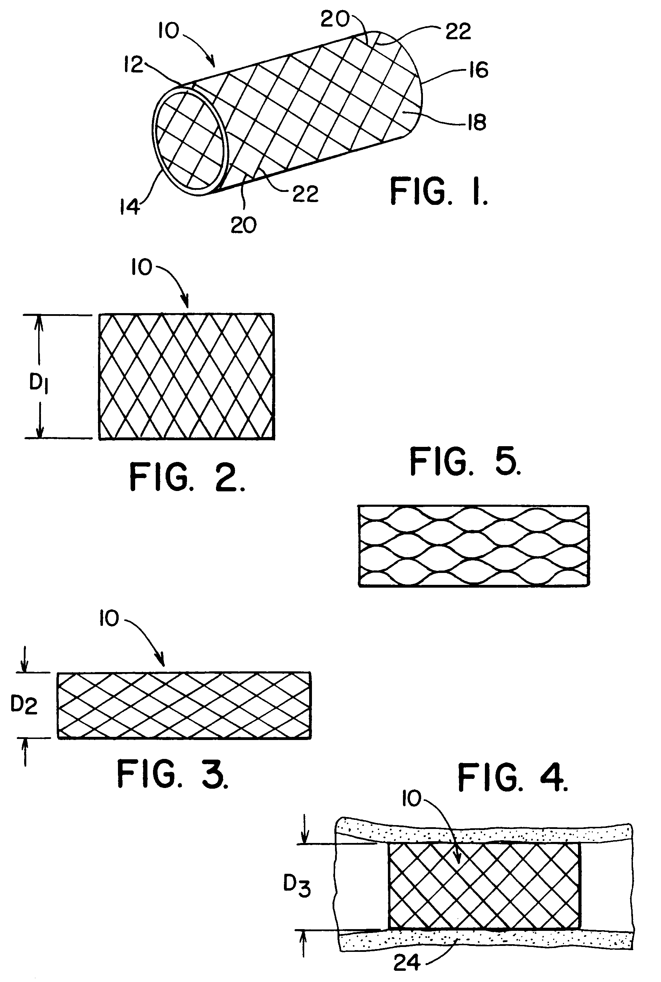

With reference to Figure 1, a self-expanding intraluminal prosthesis or stent is identified generally by numeral 10 and is seen to include a generally tubular member 12 having a pair of opposed ends 14 and 16 and a fenestrated wall surface 18. The stent of Figure 1 may be formed in a molding operation or, alternatively, may be created from a solid tube by laser or water-jet cutting the pattern of apertures so as to leave intersecting thread-like strips as at 20 and 22 therebetween.

The material from which the stent 10 is formed may be a thermoplastic having a high modulus of elasticity such that when it is subjected to inwardly directed radial forces uniformly applied over its surface, it will collapse to a lesser diameter but then spring back when the radial compressive forces are removed. A variety of medical-grade plastics are available which exhibit a high modulus of elasticity and which may be employed in fabricating the self-expanding stents of the present invention. For example, nylon or a suitable polyester may be used, but DELRIN® plastic, available through the Du Pont Corporation, has been found to be quite suitable.

Various manufacturing methods are available for fabricating the stent in accordance with this invention. Prototypes have been produced by appropriately mounting a solid tube of DELRIN® plastic on a mandrel and then, using a laser, the fenestrations or apertures are cut through the thickness dimension of the wall to form a plurality of intersecting strands creating contiguous rhombic apertures. The intersecting stands are integrally joined at their points of intersection. With no particular limitation intended, each of the individual strands 20, 22 may be 0.38 mm (0.015 in) thick in the radial direction and 0.25mm (0.010 in) wide in the circumferential direction. The laser may be computer-controlled insuring accurate spacing and precise line definition.

In a production setting, it is contemplated that the stents of the present invention may be formed in a molding operation which results in very low-cost production in comparison to the laser cutting method.

Referring next to Figures 2 through 4, at the time of manufacture, the diameter of the stent 10 is purposely oversized compared to the size of the lumen in which it is intended ultimately to be implanted. For example, it may be designed to initially have an outside diameter, $D_1$, as shown in Figure 2. Prior to insertion into the lumen of the hollow body organ to be supported, the stent of Figure 2 is radially compressed into an insertion tool and will collapse as shown in Figure 3 to exhibit a significantly lower diameter, $D_2$. When the tool and stent have been routed through the body lumen to the location where the stent is to be placed, it is released from the tool and allowed to expand to a diameter, $D_3$, which is less than diameter, $D_1$, (due to plastic deformation) and thereby provides support to the walls of the tubular organ which, in Figure 4, is identified by numeral 24.

While collapsing the stent to its smallest diameter, $D_2$, (Figure 3) results in some measure of plastic deformation, by originally over-sizing the stent as shown in Figure 2, it is capable of self-expansion to a working diameter, $D_3$, as shown in Figure 4. In fact, the stent is preferably designed such that when in position within the body organ, it will continue to exert a slight outward force against the internal walls of the body organ, thus tending to maintain the stent in position and reducing the tendency of the stent to migrate. Alternatively, appropriately disposed, radially-projecting finger-like barbs may be incorporated to resist such migration.

By loading the stent of Figure 2 into its insertion tool and thereby reducing its size to that shown in Figure 3, immediately prior to the implantation thereof, creep deformation, which is time dependent, is minimized.

In the stent shown in Figure 1, the openings are shaped like a rhombus. Good results have been achieved when the acute angles thereof are in the range of from 40° to 60° such that the corresponding obtuse angles fall into the range of from 140° to 120°. Computer analysis has shown that this shape results in a concentration of stress forces at the points of intersection of the strands where they are integrally joined. By shaping the openings as shown in the stent of Figure 5 which is the only embodiment of those described here which forms part of the present invention as claimed, the stress concentration points are significantly reduced. The apertures or openings in Figure 5 may be described as those which result when the strands defining those openings have a sinusoidal pattern and where the negative peaks of a first strand integrally join to the positive peak of an adjacent strand. Because the apertures resemble the eye opening of a human, for ease of description, they are referred to herein as eye-shaped apertures. Because the intersecting strands are integrally joined at their points of intersection, the opposed ends of the stent are free of sharp points which occur when a braided tube structure of the type disclosed in the Wallsten patent is cut to a desired length. Hence, the stent of the present invention is less traumatic to tissue at the time of its implantation.

By forming the stent of the present invention from a suitable thermoplastic material and by introducing an additive to the material, its electrical conductivity can be made comparable to that of the tissue in which the stent will become embedded. Should it become necessary or desirable to later remove the stent device, an appropriate electrosurgical instrument may be used to cut through both the involved tissue and the stent material so that the pieces resulting can be withdrawn through the body lumen in which the stent had been positioned. The fact that the conductivity of the tissue and the stent material are approximately the same results in greater uniformity and control of the electrosurgical current as the resection takes place.

## Claims

1. A stent (10) for insertion into a tubular organ for maintaining the organ patent, comprising a single-piece tubular member (12) consisting of a non-braided web or mesh formed into a closed tube of continuous section and without any overlapping longitudinal edges, having a side wall (18) exhibiting a pattern of spaced openings defined by strands (20,22), said tubular member having a single-plane fenestrated side wall, with said side wall including a pattern of regular generally eye-shaped cut-outs forming said spaced openings to allow said tubular member to be radially compressed from a larger diameter to a smaller diameter and which tubular member self-expands when the radial compressive force is removed, characterized in that said strands are intersecting strands integrally joined together at their points of intersection, the compression and later expansion not producing relative movement of said strands at said points of intersection or a significant change in axial length of said tubular member.

2. The stent according to claim 1, wherein said tubular member is formed from an electrosurgically resectable material.

3. The stent according to claim 2, wherein said electrosurgically resectable material is a thermoplastic.

4. The stent according to claim 3, wherein said resectable material is an acetal homopolymer thermoplastic resin.

5. The stent according to claim 2, wherein said electrosurgically resectable material has an electrical conductivity approximately that of body tissue.

6. The stent according to claim 1, wherein said strands have radial thickness in the range of from 1¼ to 2¼ times their width.

## Patentansprüche

1. Stent (10) zur Insertion in ein röhrenförmiges Organ, um das Organ durchgängig zu halten, der ein einstückiges, röhrenförmiges, aus einem nicht umflochtenen, zu einem geschlossenen Tubus eines fortlaufenden Bereichs gebildeten Netz oder Mesh ohne jegliche in Längsrichtung verlaufende überlappende Kante bestehendes Element (12) mit einer Seitenwand (18) mit einem Muster aus durch die Stränge (20, 22) festgelegten beabstandeten Öffnungen umfaßt, wobei das röhrenförmige Element eine eine einzelne Ebene aufweisende fenestrierte Seitenwand, die ein Muster aus regulären, im allgemeinen in Augenform ausgeformten Ausschnitten, die die beabstandeten Öffnungen bilden, so daß das röhrenförmige Element von einem größeren Durchmesser zu einem kleineren Durchmesser radial zusammengedrückt werden kann, umfaßt, aufweist und sich bei Entfernen der radialen Kompressionskraft selbst ausdehnt, dadurch gekennzeichnet, daß die Stränge sich kreuzende Stränge sind, die miteinander an ihren Kreuzungspunkten integral verbunden sind und die Kompression und spätere Expansion nicht zu einer relativen Bewegung der Stränge an den Kreuzungspunkten oder zu einer signifikanten Veränderung der Achsenlänge des röhrenförmigen Elements führen.

2. Stent nach Anspruch 1, wobei das röhrenförmige Element aus einem elektrochirurgisch resektierbaren Material gebildet ist.

3. Stent nach Anspruch 2, wobei das elektrochirurgisch resektierbare Material ein thermoplastisches Material ist.

4. Stent nach Anspruch 3, wobei das resektierbare Material ein thermoplastisches Acetalhomopolymerharz ist.

5. Stent nach Anspruch 2, wobei das elektrochirurgisch resektierbare Material eine elektrische Leitfähigkeit aufweist, die derjenigen des Körpergewebes etwa gleicht.

6. Stent nach Anspruch 1, wobei die Stränge eine radiale Dicke im Bereich des 1¼- bis 2¼fachen ihrer Breite aufweisen.

## Revendications

1. Dilatateur (10) destiné à être introduit dans un organe tubulaire pour maintenir l'organe inobstrué, comprenant un élément tubulaire monobloc (12) consistant en une trame ou un treillis non tressé, réalisé dans un tube fermé de section continue et sans bords longitudinaux qui se chevauchent, comportant une paroi latérale (18) présentant un motif de trous distance les uns des autres, délimités par des brins (20, 22), ledit élément tubulaire ayant une paroi latérale située dans un plan unique, comportant des fenêtres, ladite paroi latérale comprenant un motif de découpes régulières, sensiblement en forme d'oeil, formant lesdits trous espacés pour permettre audit élément tubulaire d'être comprimé radialement d'un grand diamètre à un diamètre plus petit et ledit élément tubulaire subissant une auto-expansion lorsque la force radiale de compression est supprimée, caractérisé en ce que lesdits brins sont des brins qui forment des points d'intersection, qui sont reliés monobloc à leurs points d'intersection, la compression et l'expansion ultérieure ne produisant aucun mouvement relatif desdits brins auxdits points d'intersection ni une modification notable de la longueur axiale dudit élément tubulaire.

2. Dilatateur selon la revendication 1, dans lequel ledit élément tubulaire est réalisé en une matière capable de subir une résection par voie électrochirurgicale.

3. Dilatateur selon la revendication 2, dans lequel ladite matière capable de subir une résection par voie électrochirurgicale est une matière thermoplastique.

4. Dilatateur selon la revendication 3, dans lequel ladite matière capable de subir une résection est une résine thermoplastique à base d'un homopolymère d'acétal.

5. Dilatateur selon la revendication 2, dans lequel ladite matière capable de subir une résection par voie électrochirurgicale a une conductivité électrique qui est approximativement celle du tissu du corps.

6. Dilatateur selon la revendication 1, dans lequel lesdits brins ont une épaisseur radiale de l'ordre de 1¼ à 2¼ fois leur largeur.

FIG. 1.

FIG. 2.

FIG. 3.

FIG. 4.

FIG. 5.